# EUROPÄISCHE PATENTSCHRIFT

(11) **EP 1 928 836 B1**
(45) Veröffentlichungstag und Bekanntmachung des Hinweises auf die Patenterteilung: **15.07.2015**
(21) Anmeldenummer: 06805671.2
(22) Anmeldetag: 08.09.2006
(51) Int. Cl.: C07D 217/14, A61K 31/47, A61P 9/12

(54) **SUBSTITUIERTE 4-PHENYLTETRAHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 4-PHENYLTETRAHYDROISOQUINOLINES, METHOD OF PRODUCING THEM, THEIR USE AS MEDICAMENT, AND ALSO MEDICAMENT CONTAINING THEM
4-PHENYLTETRAHYDROISOQUINOLEINES SUBSTITUEES, PROCEDE DE PRODUCTION, LEUR UTILISATION COMME MEDICAMENT ET MEDICAMENT LES CONTENANT

(30) Priorität: 20.09.2005 DE 102005044817
(43) Veröffentlichungstag der Anmeldung: 11.06.2008
(73) Patentinhaber: SANOFI, 75008 Paris (FR)
(72) Erfinder: LANG, Hans-Jochen, 65719 Hofheim (DE)
(74) Vertreter: Michalski Hüttermann & Partner Patentanwälte mbB
(86) Internationale Anmeldenummer: PCT/EP2006/008770
(87) Internationale Veröffentlichungsnummer: WO 2007/033773

(56) Entgegenhaltungen:
- WO-A-01/79186
- WO-A-03/048129
- WO-A2-03/055880
- DE-A1- 10 312 963

## Beschreibung

Die Erfindung betrifft substituierte 4-Phenyltetrahydroisochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion und bei Atemstörungen wie Schnarchen oder Schlafapnoen einsetzen.

Die Erfindung betrifft Verbindungen der Formel I 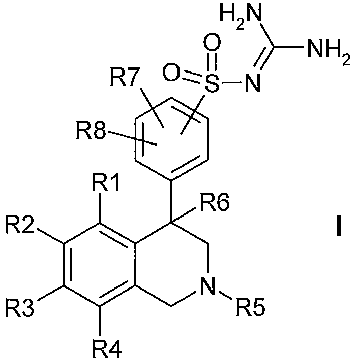 worin bedeuten:
- R1 und R3: Wasserstoff;
- R2 und R4: unabhängig voneinander Wasserstoff, F, Cl, NH₂, NHCH₃ oder N(CH₃)₂;
- R5: Wasserstoff, Methyl, Ethyl oder Cyclopropyl;
- R6: Wasserstoff oder Methyl;
- R7 und R8: Wasserstoff;
sowie deren pharmazeutisch verträgliche Salze und Trifluoracetate.

Speziell bevorzugt wird N-Diaminomethylen-4-(6,8-dichloro-2-methyl-1,2,3,4-tetrahydro-isochinolin-4-yl)-benzolsulfonamid sowie dessen pharmazeutisch verträglichen Salze und Trifluoracetate.

In einer Ausführungsform sind Verbindungen der Formel I bevorzugt, in denen R1 und R3 Wasserstoff sind und R2 und R4 unabhängig voneinander Wasserstoff, F, Cl, NH₂, NHCH₃ oder N(CH₃)₂, beispielsweise Cl. In einer Ausführungsform sind Verbindungen der Formel I bevorzugt, in denen R2 und R4 nicht Wasserstoff sind.

In einer weiteren Ausführungsform sind Verbindungen der Formel I bevorzugt, in denen R5 durch Wasserstoff, Methyl, Ethyl oder Cyclopropyl beschrieben wird, beispielsweise Methyl.

In einer weiteren Ausführungsform sind Verbindungen der Formel I bevorzugt, in denen R6 durch Wasserstoff oder Methyl beschrieben wird.

In einer weiteren Ausführungsform sind Verbindungen der Formel I bevorzugt, in denen R7 und R8 Wasserstoff sind.

Enthalten die Verbindungen der Formel I ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen.

Die vorliegende Erfindung umfasst alle möglichen tautomeren Formen der Verbindungen der Formel I.

Die vorliegende Erfindung umfasst weiterhin Derivate der Verbindungen der Formel I, zum Beispiel Solvate, wie Hydrate und Alkoholaddukte, Ester, Prodrugs und andere physiologisch akzeptablen Derivate der Verbindungen der Formel I, sowie aktive Metaboliten der Verbindungen der Formel I. Die Erfindung umfasst ebenfalls alle Kristallmodifikationen der Verbindungen der Formel I.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Da Verbindungen der Formel I stets wenigstens eine basische Gruppe enthalten, können sie auch in Form ihrer physiologisch verträglichen Säureadditionssalze z. B. mit folgenden Säuren hergestellt werden: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate (diese Gruppe entspricht auch den physiologisch akzeptablen Anionen); aber auch Trifluoracetate.

Gegenstand der Erfindung sind auch die im folgenden beschriebenen Verfahren zur Herstellung von Verbindungen der Formel I.

Die hier beschriebenen Verbindungen der Formel I lassen sich herstellen durch Chlorsulfonierung von Verbindungen der Formel VIII mittels dem Fachmann bekannter Verfahren mit anschließender Umsetzung mit Guanidin nach dem Fachmann bekannten Verfahren (wie z.B. in Synthetic Communications, 33(7), 1073; 2003 beschrieben).

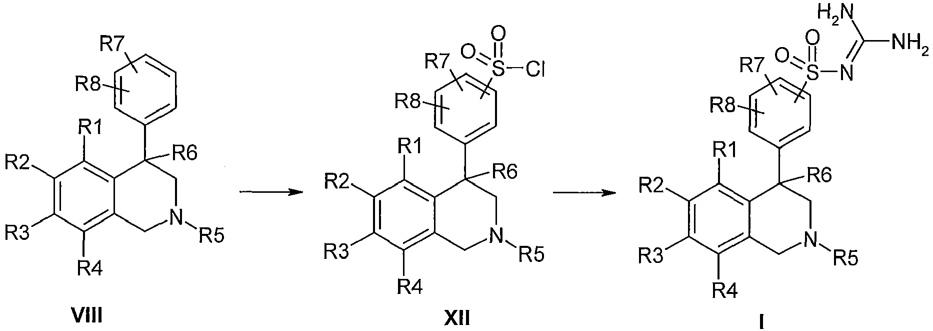

Die nach der Chlorsulfonierung erhaltene Zwischenstufe der Formel XII braucht dabei nicht isoliert zu werden, sondern kann direkt weiter mit Guanidin umgesetzt werden.

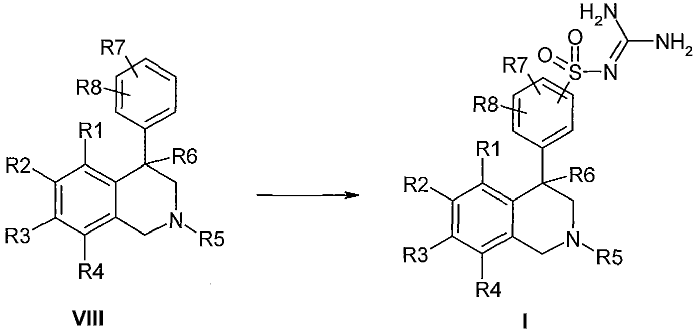

Die Ausgangsverbindungen der Formel VIII können wie folgt hergestellt werden:

Durch Reduktion der Carbonylgruppierung in Verbindungen der Formel VI, zum Beispiel mit Natriumborhydrid, und anschließende säure- oder basenkatalysierte Cyclisierung der entstandenen Alkohole der Formel VII (vgl. Tetrahedron Lett. 1989, 30, 5837; Org. Prep. Proced. Int. 1995, 27, 513) können Tetrahydroisochinoline der Formel VIIIa nach dem Fachmann bekannten Verfahren hergestellt werden, wobei R1 bis R8 die oben angegebene Bedeutung besitzen.

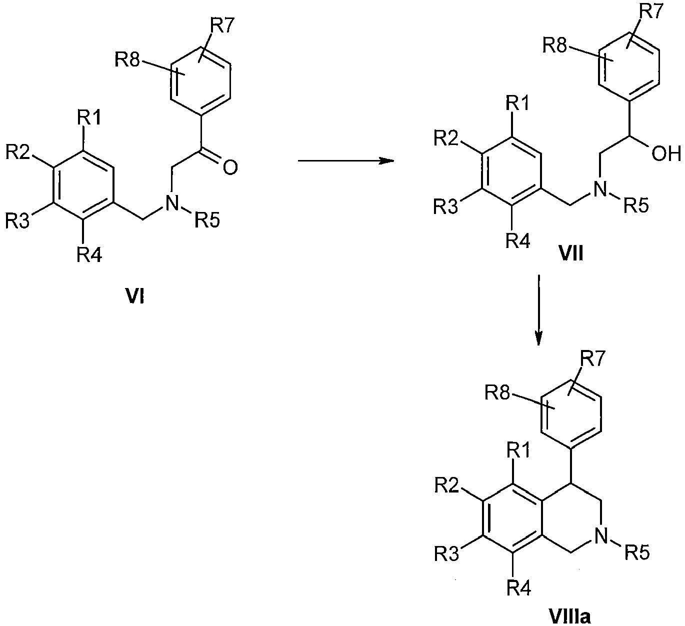

Zur Herstellung alkylverzweigter Verbindungen der Formel I, in denen R6 nicht Wasserstoff ist, können die entsprechenden Diphenylessigsäureester der Formel IX in alpha-Stellung mit R6 nach bekannten Methoden alkyliert werden. Die Verbindungen der Formel X können durch Standardverfahren in die entsprechenden Amide der Formel XI überführt werden, welche in einer Pictet-Spengler-analogen Reaktion in die gewünschten Tetrahydroisochinoline der Formel VIIIb überführt werden (vgl. Tetrahedron 1987, 43, 439; Chem. Pharm. Bull. 1985, 33, 340), wobei R1 bis R8 wie oben definiert sind und LG einer bei Alkylierungen gängigen Fluchtgruppe, wie beispielsweise Bromid, Chlorid, Tosylat oder Mesylat, entspricht.

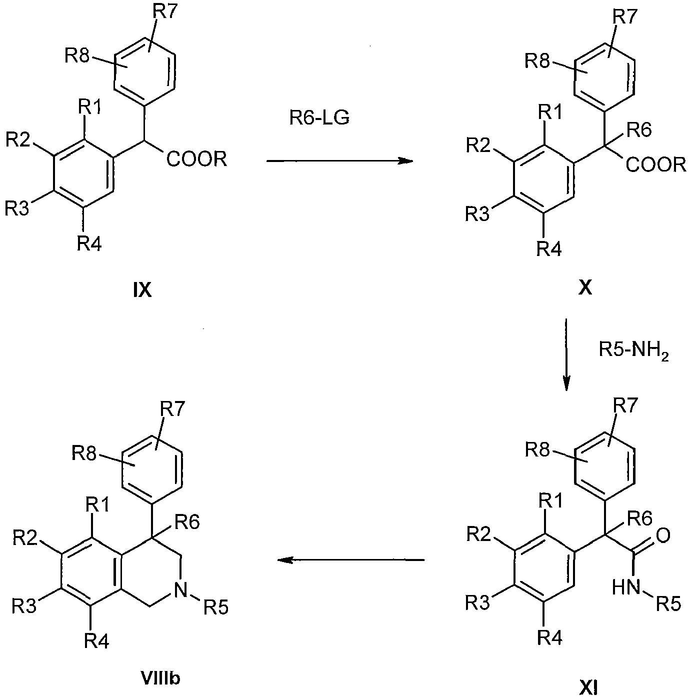

Die oben eingesetzten Verbindungen der Formel VI werden vorzugsweise aus Benzylaminen der Formel IV in dem Fachmann bekannter Weise und den entsprechenden Amino-substituierten alpha-Bromacetophenon-Verbindungen der Formel V hergestellt,
wobei R1 bis R8 wie oben definiert sind,

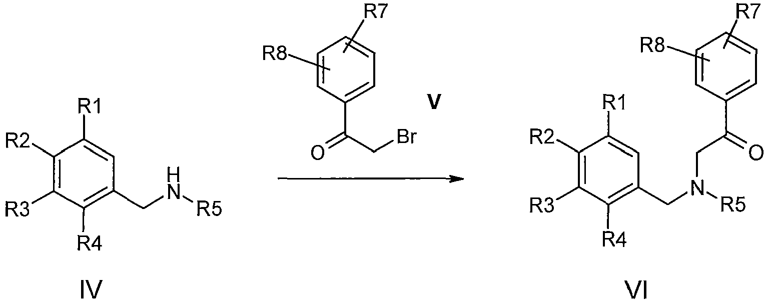

Die alpha-Bromacetophenon-Verbindungen der Formel V lassen sich in literaturbekannten Verfahren aus den entsprechenden Acetophenon-Vorläufern durch Bromierung gewinnen.

Die Benzylamin-Vorläufer der Formel IV können, falls nicht käuflich erhältlich, nach dem Fachmann bekannten Standardverfahren aus den entsprechenden Benzylhalogeniden, beispielweise Benzylchloriden oder -bromiden, der Formel II und den entsprechenden Aminen R5-NH₂ synthetisiert werden,
wobei R1 bis R5 wie oben definiert sind und X F, Cl, Br oder I, insbesondere Cl oder Br ist.

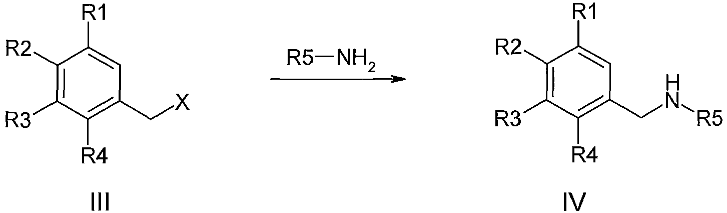

Alternativ sind Verbindungen der Formel IV auch durch reduktive Aminierung eines Aldehyds der Formel IIIa nach dem Fachmann bekannten Standardverfahren zugänglich,
wobei R1 bis R5 wie oben definiert sind.

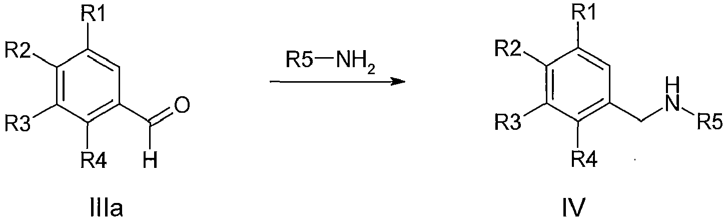

Die Verbindungen der Formeln III und IIIa, IX und R6-LG und R5-NH₂ sind käuflich erhältlich oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen. Weiterhin sind die Verbindungen der Formel I auch hervorragende Inhibitoren des Natrium-Wasserstoffaustauschers vom Subtyp 5 (NHE5).

Die bisher bekannten NHE3-Inhibitoren leiten sich beispielsweise von Verbindungen des Acylguanidin-Typs (EP825178), Norbornylamin-Typs (WO0144164), 2-Guanidino-chinazolin-Typs (WO0179186) oder Benzamidin-Typs (WO0121582, WO0172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde.

Tetrahydroisochinoline als Inhibitoren des Natrium-Wasserstoffaustauschers vom Subtyp 3 (NHE3) werden bereits in den Patentanmeldung WO03048129, WO2004085404 und den deutschen Anmeldungen Nr. 102004046492.8 und 102005001411.9 beschrieben. In der Patentanmeldung WO03055880 ist die verwandte Verbindungsklasse der Tetrahydroisochinolinium-Salze als NHE3-Inhibitoren beschrieben. Überraschend wurde nun gefunden, dass die hier beschriebenen Verbindungen der Formel I ebenfalls potente Inhibitoren des NHE3 und des NHE5 darstellen und dabei vorteilhafte pharmakologische und pharmakokinetische Eigenschaften besitzen.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603). Der NHE5 ist nur in Neuronen exprimiert und daher gehirnspezifisch (Am. J. Physiol. Cell. Physiol. 281: C1146-C1157, 2001).
Aufgrund ihrer NHE-inhibitorischen Eigenschaften eignen sich die Verbindungen der Formel I zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.
Die Verbindungen der Formel I können auch zur Behandlung und Prävention von Krankheiten eingesetzt werden, wobei der NHE nur partiell gehemmt wird, beispielsweise durch Verwendung einer niedrigeren Dosierung.

Die Verwendung der erfindungsgemäßen Verbindungen betrifft die Prävention und die Behandlung akuter und chronischer Krankheiten in der Veterinär- und in der Humanmedizin.

Infolge Ihrer pharmakologischen Wirkungen sind die Verbindungen der Formel I insbesondere dazu geeignet, zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so dass eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik auftreten, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist deren kombinierte Verabreichung mit Verbindungen der Formel I geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.
Die Verbindungen der Formel I eignen sich insbesondere zur Verbesserung der Therapie mit Arzneimitteln, die eine unerwünschte cardiotoxische Komponente besitzen.

Generell können die hier beschriebenen NHE Inhibitoren in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie mit anderen NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüber hinaus können sie die nach Ischämie und Reperfusion stattfindende überschießende Freisetzung von Entzündungs- und Gerinnungsmediatoren, insbesondere des von Willebrand Faktors und thrombogener Selectin-Proteine, hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-RezeptorAntagonisten, Phosphodiesterase-Inhibitoren, Faktor-Vlla-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel I zur Prävention und zur Behandlung des Bluthochdrucks und von Herz-Kreislauferkrankungen. Dabei können sie allein oder mit einem geeigneten Kombinationspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch ß-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren weiterer Kaliumkanäle, wie des Kv1.5 usw.

Infolge ihrer antiphlogistischen Wirkung können erfindungsgemäße NHE-Inhibitoren als Antiinflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.
Dabei kommt eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, in Betracht. Die Verbindungen der Formel I eignen sich somit zur Prävention altersbedingter Gewebsveränderungen und zur Gesunderhaltung und Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen.
Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.
Die genannten Verbindungen finden daher vorteilhaft Verwendung allein oder in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

Der Begriff Demenz bezeichnet einen Verfall der geistigen Leistungsfähigkeit. Man versteht darunter vor allem die Abnahme von Gedächtnisleistung und Denkvermögen. Die Altersdemenz oder senile Demenz bezeichnet einen fortschreitenden, erworbenen geistigen Abbau bei Personen höheren Alters, welcher auf strukturelle und/oder metabolische Abnormitäten im Zentralnervensystem zurückzuführen ist. Ungefähr 7% der Bevölkerung über 65 Jahre leidet an einer unterschiedlich ausgeprägten Demenz. Die Ursachen für Demenzen sind unterschiedlich: Die Alzheimer-Krankheit stellt mit bis zu 50% die häufigste Form dar, gefolgt von vaskulären Demenzen, wie Multi-Infarkt-Demenz, und Kombinationen dieser beiden Formen. Viel seltenere Ursachen sind Tau-Mutationen, Prionen-Erkrankungen, Polyglutamin-Expansions-Erkrankungen, wie Chorea Huntington und spinocerebelläre Ataxien, und Parkinson. Weiterhin sind auch sekundäre Demenzen nach und/oder bei Infekten (z.B. mit HIV), Hirntraumen, Hirntumoren oder Intoxikationen (z.B. mit Alkohol) bekannt.

Das Konzept der Gedächtniskonsolidierung beruht darauf, dass neugebildete Gedächtnisinhalte sich über die Zeit stabilisieren können und dadurch weniger empfänglich für Störungen durch neue Informationen und Fehlfunktionen des Gehirns werden. Mit Hilfe des vorherrschenden zellulären Modells der Langzeitpotenzierung (LTP) können wesentliche Aspekte und Mechanismen der Gedächtnisbildung und - konsolidierung untersucht werden (Neuroscientist. 9: 463-474. 2003; Brain Res Brain Res Rev. 45: 30-37, 2004; Physiol Rev. 84: 87-136, 2004).

Eine der wichtigsten Hirnregionen, in denen Informationen gespeichert und verarbeitet werden, ist die Hippocampusformation. Es ist lange bekannt, dass im Hippocampus bestimmte Muster elektrischer Stimulation (Tetanisierung) zu Veränderungen der synaptischen Effizienz führen (Bliss und Lomo, J Physiol. 232: 331-356, 1973), die heute als 'Langzeitpotenzierung' oder 'LTP' bezeichnet werden, und die nachfolgend in anderen Gehirngebieten verschiedenster Säugetiere sowohl in vitro als auch in vivo bestätigt wurden. Die LTP wird heute als wichtige Komponente des neuronalen Mechanismus angesehen, der dem Lernen und dem Gedächtnis zu Grunde liegt. Weiterhin ist bekannt, dass eine schwache LTP mit dem Kurzzeitgedächtnis, und eine starke LTP mit dem Langzeitgedächtnis korreliert (J Neurosci. 20: 7631-7639, 2000; Proc Natl Acad Sci USA. 97: 8116-8121, 2000).

Der Hippokampus spielt eine zentrale Rolle bei episodischen, räumlichen und deklarativen Lern- und Gedächtnisvorgängen, er ist essentiell für die räumliche Orientierung und Erinnerung von räumlichen Strukturen und spielt eine wichtige Rolle bei der Kontrolle autonomer und vegetativer Funktionen (McEwen 1999, Stress and hippocampal plasticity, Annual Review of Neuroscience 22: 105-122). Bei Demenzerkrankungen des Menschen sind zumeist die Lern- und Gedächtnisprozesse gestört, an denen der Hippocampus beteiligt ist. Ähnliches wurde auch in Tierexperimenten an anderen Säugern gezeigt.
So konnte gezeigt werden, dass alte Mäuse im Vergleich zu jungen Mäusen Defizite im räumlichen Gedächtnis und in der LTP aufweisen, und dass Substanzen, die die
LTP verbesserten, gleichzeitig die Gedächtnisdefizite verminderten (Bach et al. 1999, Age-related defects in spatial memory are correlated with defects in the late phase of hippocampal long-term potentiation in vitro and are attenuated by drugs that enhance the cAMP signaling pathway. Proc Natl Acad Sci USA. 27;96:5280-5; Fujii & Sumikawa 2001, Acute and chronic nicotine exposure reverse age-related declines in the induction of long-term potentiation in the rat hippocampus. Brain Res. 894:347-53, Clayton et al. 2002, A hippocampal NR2B deficit can mimic age-related changes in long-term potentiation and spatial learning in the Fischer 344 rat. J Neurosci.22:3628-37).
An transgenen Tieren und durch die Applikation von Beta-Amyloidpeptiden konnte in vivo und in vitro gezeigt werden, dass die Peptide LTP verschlechtern bzw. deren Aufrechterhaltung stören (Ye & Qiao 1999, Suppressive action produced by betaamyloid peptide fragment 31-35 on long-term potentiation in rat hippocampus is N-methyl-D-aspartate receptor-independent: it's offset by (-)huperzine A. Neurosci Lett.275:187-90. Rowan et al 2003, Synaptic plasticity in animal models of early Alzheimer's disease. Philos Trans R Soc Lond B Biol Sci. 358: 821-8, Gureviciene et al. 2004, Normal induction but accelerated decay of LTP in APP + PS1 transgenic mice. Neurobiol Dis15:188-95). Die Beeinträchtigung der LTP bzw. der Gedächtnisfunktionen konnte durch Rolipram und Cholinesteraseinhibitoren, wie sie auch in der humanen Alzheimer-Therapie eingesetzt werden, korrigiert werden (Ye & Qiao 1999, Gong et al. 2004, Persistent improvement in synaptic and cognitive functions in an Alzheimer mouse model after rolipram treatment.J Clin Invest. 114:1624-34.)

Es ist somit zu erwarten, dass Substanzen, die die LTP verbessern, auch eine positive Wirkung bei Erkrankungen haben, die mit kognitiven Störungen und Demenz einhergehen.

Überraschend wurde gefunden, dass Inhibitoren des zellulären NHE5 die LTP verstärken. Damit ist eine gedächtnisverbessernde Wirkung des Inhibitors bei Demenzerkrankungen wie Alzheimer und Alzheimer-ähnlichen Formen der Demenz zu erwarten. Der Einsatz eines NHE5 Inhibitors hat gegenüber den bei diesen Erkrankungen bisher eingesetzten Wirkstoffen, wie Acetylcholinesterasehemmern, den Vorteil, dass systemische Wirkungen voraussichtlich gering sein werden oder fehlen, da der NHE5 nur in Neuronen exprimiert ist und daher gehirnspezifisch ist (Am. J. Physiol. Cell. Physiol. 281: C1146-C1157, 2001).

Daher eignen sich NHE5 Inhibitoren zur Behandlung von neurodegenerativen Krankheiten, Gedächtnisstörungen und Demenzerkrankungen wie Altersdemenz, Alzheimer, vaskulären Demenzen, wie zum Beispiel Multi-Infarkt-Demenz, Kombinationen von Alzheimer und cereborvaskulären Erkrankungen, Tau-Mutationen, Prionen-Erkrankungen, Polyglutamin-Expansions-Erkrankungen, wie zum Beispiel Chorea Huntington und spinocerebelläre Ataxien, und Parkinson, sowie zur Gedächtnisverbesserung. Weiterhin eignen sich NHE5 Inhibitoren zur Behandlung sekundärer Demenzen nach und/oder bei Infektionen, wie zum Beispiel mit HIV, Hirntraumen, Hirntumoren oder Intoxikationen, wie zum Beispiel mit Alkohol.

Die Erfindung betrifft weiterhin die Verwendung der Verbindungen der Formel I und deren pharmazeutisch verträgliche Salze zur Verwendung als Medikament.

Die Erfindung betrifft auch Heilmittel für die humane, veterinäre oder phytoprotektive Anwendung enthaltend eine wirksame Menge einer Verbindung der Formel I und / oder eines pharmazeutisch verträglichen Salzes davon, ebenso wie Heilmittel für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.
Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.-Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

### Liste der verwendeten Abkürzungen:

- AMPA: durch α-Amino-3-hydroxy-5-methylisoxazol-4-propionat aktivierbare rezeptorgekoppelte Kanäle
- CA 1: CA = Cornu ammonis (Ammonshorn), CA Region 1 im Hippokampus
- EE: Ethylacetat
- EPSP: Exzitatorisches postsynaptisches Potential
- ES⁺: electron spray
- HEP: n-Heptan
- Konz. NH₃: gesättigte wäßrige NH₃-Lösung
- LTP: Langzeitpotenzierung
- LTP1: frühe LTP (Phase der LTP)
- MeOH: Methanol
- mp: Schmelzpunkt
- MS: Mass Spectroscopy
- NMDA: durch N-Methyl-D-Aspartat aktivierbare rezeptorgekoppelte Kanäle
- RT: Raumtemperatur
- STP: Short-term potentiation (Phase der LTP)
- THF: Tetrahydrofuran

### Beispiel 1: N-Diaminomethylen-4-(6,8-dichloro-2-methyl-1,2,3,4-tetrahydro-isochinolin-4-yl)-benzolsulfonamid, dihydrochlorid

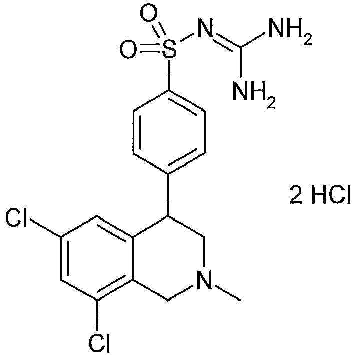

0.36g Guanidin werden unter Argon in 30 ml wasserfreiem THF suspendiert und 0.40g 4-(6,8-Dichloro-2-methyl-1,2,3,4-tetrahydro-isochinolin-4-yl)-benzolsulfonylchlorid (WO2003048129) zugegeben. 24 h wurde bei RT gerührt, dann das THF abdestilliert. Der Rückstand wurde mit 10 ml Wasser versetzt und der Niederschlag abfiltriert. Mit 10 ml Wasser wurde gewaschen und im Vakuum getrocknet. Der Feststoff wurde dann in 10 ml EE suspendiert und mit 10 ml einer gesättigten Lösung von HCl in Diethylether versetzt. Die flüchtigen Bestandteile wurden im Vakuum entfernt, der Rückstand in 10 ml EE suspendiert und 5 h bei RT gerührt. Der Niederschlag wurde dann abfiltriert und im Vakuum getrocknet. Und man erhielt 0.45 g , mp 140°C (Zersetzung).
R_{f} (EE/HEP/CH₂Cl₂/MeOH/konz. NH₃ = 10:5:5:5:1) = 0.30 MS (ES⁺) : 412

### Pharmakologische Daten:

### NHE3 und NHE5-Testbeschreibung:

In diesem Test wurde die Erholung des intrazellulären pH-Wertes (pHᵢ) von LAP1-Zellen, die unterschiedliche Subtypen des Natrium-Protonen-Austauschers (NHE) stabil exprimieren, nach einer Ansäuerung ermittelt. Diese Erholung setzt bei funktionsfähigem NHE auch unter bicarbonatfreien Bedingungen ein. Dazu wurde der pHᵢ mit dem pH-sensitiven Fluoreszenzfarbstoff BCECF (Molecular Probes, Eugene, OR, USA, eingesetzt wird die Vorstufe BCECF-AM) bestimmt. Die Zellen wurden zunächst mit BCECF (5 µM BCECF-AM) in NH₄Cl-Puffer (NH₄Cl-Puffer: 115 mM CholinCl, 20 mM NH₄Cl, 5 mM KCl, 1 mM CaCl₂, 1 mM MgCl₂, 20 mM Hepes, 5 mM Glucose, mit 1 M KOH wird ein pH von 7,4 eingestellt) inkubiert. Die intrazelluläre Ansäuerung wurde durch Waschen der in NH₄Cl-Puffer inkubierten Zellen mit NH₄Cl-freien Puffer (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M KOH wird ein pH von 7,4 eingestellt) induziert. Nach dem Waschvorgang wurden 90 µl des NH₄Cl-freien Puffer auf den Zellen belassen. Die pH-Erholung wurde durch die Zugabe vom 90 µl Na⁺-haltigem Puffer (133,8 mM NaCl, 4,7 mM KCl, 1,25 mM CaCl₂, 1,25 mM MgCl₂, 0,97 mM Na₂HPO₄, 0,23 mM NaH₂PO₄, 10 mM Hepes, 5 mM Glucose, mit 1 M NaOH wird ein pH von 7,4 eingestellt) im Meßgerät (FLIPR, "Fluorometric Imaging Plate Reader", Molecular Devices, Sunnyvale, Ca., USA) gestartet. Die BCECF-Fluoreszenz wurde bei einer Anregungswellenlänge von 498 nm und dem FLIPR-Emissionsfilter 1 (Bandpass von 510 bis 570 nm) bestimmt. Die nachfolgenden Fluoreszenzänderungen als Maß der pH-Erholung wurden bei NHE3 und NHE5 für zwei Minuten registriert. Für die Berechnung der NHE-inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer (s.o.), für die Bestimmung des 0%-Wertes in Na⁺-freien Puffer (s.o.) inkubiert. Die zu testenden Substanzen wurden in Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms XLFit (idbs, Surrey, UK) der IC₅₀-Wert der jeweiligen Substanz für die einzelnen NHE-Subtypen berechnet.

| | NHE3 IC₅₀ [µM] | NHE5 IC₅₀ [µM] |
|---|---|---|
| Beispiel 1 | 0.035 | 0.37 |

### Testbeschreibung: Langzeit-Experimente an Hippokampusschnitten (in vitro)

### Experimenteller Ansatz

Die LTP in der CA 1 Region ist die am besten charakterisierte LTP in vitro. Die Schichtung und Eingangsstruktur dieser Region erlaubt Feldpotentialmessungen über mehrere Stunden in vitro. In den NHE Studien wurde ein an den Thetarhythmus angelehnten schwachen Tetanus benutzt, der eine frühe LTP induziert, die innerhalb von drei Stunden auf den Ausgangswert zurückgeht (Journal of Neuroscience, 18(16), 6071 (1998); Eur J Pharmacol. 502: 99-104, 2004). Es wurde kürzlich bestätigt, dass eine zunehmende Zahl von Thetafrequenzimpulsfolgen eine LTP von zunehmendem Ausmaß und Persistenz induziert (J Neurophysiol. 88:249-255, 2002), d.h. dass ein einzelner schwacher Stimulus nicht den maximal erzielbare gesättigten Typ der LTP, sondern eine ungesättigte LTP induziert. Sowohl Ausmaß (Behnisch, Reymann et al., Neurosci. Lett. 1998, 253(2): 91-94) als auch Persistenz (z.B. Neuropeptides 26: 421-427, 1994) dieser LTP kann durch Substanzen verbessert oder verschlechtert werden. Die frühe LTP, die wir in unseren Untersuchungen erzeugen, ist ebenfalls ungesättigt. Man kann damit eine substanzinduzierte Verbesserung bzw. Verschlechterung der frühen LTP feststellen. Die untersuchte frühe LTP setzt sich aus der STP Komponente, die bekanntermaßen etwa 30 Minuten andauert (Nature 335: 820-824, 1988), und der LTP 1 Komponente, die gewöhnlich in den ersten 1 - 2 Stunden nach LTP Induktion (Learn Mem. 3: 1-24, 1996) andauert.
Die kurze (30-60 Minuten) Aufzeichnung der Ausgangswerte vor dem Tetanus erlaubt die Untersuchung von frühen Effekten der zu untersuchenden Substanz auf die normale, unstimulierte synaptische Übertragung. Da die hauptsächlichen exzitatorischen Synapsen glutamaterg sind (J Clin Neurophysiol. 9: 252-263, 1992), d.h. das monosynaptische Feld EPSP weitestgehend durch AMPA und nur zu einem wesentlich geringeren Anteil von NMDA Rezeptoren bestimmt wird, testet man damit indirekt gleichzeitig eine Wirkung auf die glutamaterge Transmission.

### Methode: Langzeit-Experimente an Hippokampusschnitten (in vitro)

### Art der Tiere:

Alter: 7-8 Wochen
Stamm: Wistar (Shoe Wist, Shoe)
Geschlecht: männlich
Zucht: Harlan Winkelmann GmbH, künstliches Licht (6-18 Uhr) und Tagesrhythmus

### Präparation:

Betäubung: Schlag mit Eisenstab auf Genick
Tötung: Dekapitation

### Hirnfreilegung: Öffnen der Schädeldecke durch Aufschneiden der Sagittalnaht des Schädels von dorsal nach ventral

Freilegung des Hippokampus: Das Hirn wurde zwischen den Hemisphären eingeschnitten und beginnend mit der rechten Hemisphäre der Hippokampus mit einem stumpfen Gegenstand herausgehoben.

Herstellung der Schnitte: Der freigelegte Hippokampus wurde auf einen Kühlblock mit feuchtem Filterpapier überführt und die überschüssige Feuchtigkeit mit Hilfe eines anderen Filterpapiers abgesaugt. Dieser so auf den Kühlblock befestigte Hippokampus wurde auf den Chopper gestellt und horizontal verdreht, bis der Hippokampus in einem entsprechenden Winkel zur Schneideklinge lag.

Schnittwinkel: Um die laminare Struktur des Hippokampus aufrecht zu erhalten war es notwendig, den Hippokampus in einen Winkel von etwa 70 Grad im Verhältnis zur Schnittklinge zu schneiden (Chopper).

Schnitt: In Abständen von 400 µm wurde der Hippokampus zerschnitten. Die Schnitte wurden mit Hilfe eines sehr weichen, stark benetzten Pinsels (Marderhaar) von der Klinge genommen und in ein Glasgefäß mit carbogenisierter gekühlter Nährlösung überführt. Die gesamte Präparationsdauer dauerte nicht länger als 5 min.

### Aufbewahrung der Schnitte:

Tauchschnitt: Die Schnitte lagen in einer temperierten Kammer (33° C) unter einem Flüssigkeitsspiegel von 1-3 mm. Die Durchflußrate betrug 2,5 ml/min. Die Vorbegasung erfolgte unter geringen Überdruck (etwa 1 atm) sowie über eine Mikrokanüle in der Vorkammer. Die Schnittkammer war mit der Vorkammer so verbunden, daß eine Minizirkulation aufrechterhalten werden konnte. Als Antrieb der Minizirkulation wurde das durch die Mikrokanüle ausströmende Carbogen eingesetzt.

Schnittadaptation: Die frisch präparierten Hippokampusschnitte wurden mindestens 1 h bei 33 °C in der Schnittkammer adaptiert.

### Bestimmung der Testreizstärke:

Reizstärke: fEPSP: 30% des EPSP-Maximums

### Messung der fokalen Potentiale

Stimulation: Mit Hilfe einer monopolaren Stimulationselektrode, die aus lackiertem Edelstahl bestand und eines stromkonstanten, biphasischen Reizgenerators (WPI A 365), wurden lokal die Schaffer-Kollateralen erregt (Spannung: 1-5 V, Impulsbreite einer Polarität 0.1 ms, Gesamtimpuls 0.2 ms).

Messung: Mit Hilfe von Glaselektroden (Borosilikatglas mit Filament, 1-5 MOhm, Durchmesser: l.5 mm, Spitzendurchmesser: 3-20 µm), die mit normaler Nährlösung gefüllt waren, wurden aus dem Stratum radiatum die exzitatorischen postsynaptischen Potentiale (fEPSP) registriert. Die Messung der Feldpotentiale geschah gegenüber einer chlorierten Referenzelektrode aus Silber, die sich am Rande der Schnittkammer befand, mit Hilfe eines Gleichspannungsverstärker. Das Filtern der Feldpotentiale erfolgte über einen Low-Pass Filter (5 kHz).

Ermittlung der Feldpotentialwerte: Für die statistische Analyse der Experimente wurde der Anstieg (slope) der fEPSPs (fEPSP-Anstieg) ermittelt. Die Aufnahme, Analyse und Steuerung des Experimentes erfolgte mit Hilfe eines Softwareprogrammes (PWIN), welches in der Abteilung Neurophysiologie entwickelt wurde. Die Mittelwertbildung der fEPSP-Anstiegswerte zu den jeweiligen Zeitpunkten und die Konstruktion der Diagramme erfolgte mit Hilfe der Software EXCEL, wobei ein entsprechendes Makro die Aufnahme der Daten automatisierte.

### Nährmedium (Ringerlösung):

| Substanz | in mM | für 1 l in g |
|---|---|---|
| NaCl | 124 | 7.248 |
| KCl | 4.9 | 0.356 |
| MgSO4* 7H2O | 1.3 | 0.321 |
| CaCl2+ Wasser- frei | 2.5 | 0.368 |
| KH2PO4 | 1.2 | 0.164 |
| NaHCO3 | 25.6 | 2.152 |
| Glucose | 10 | 1.802 |
| Osmolarität in mOsm | 330 | |
| PH | 7.4 | |

Beispiel 1 wurde in DMSO gelöst und für die Experimente mit Ringerlösung auf die Endkonzentration verdünnt (Endkonzentration DMSO 0.01%).

### Versuchsablauf:

In den Kontrollexperimenten wurde zunächst für 60-120 Minuten die basale synaptische Transmission registriert. Anschließend wurden im Abstand von 200 ms viermal zwei Doppelpulsen mit einem Interpulsabstand der Doppelpulse von 10 ms und einer Breite der Einzelpulse von 0,2 ms (schwacher Tetanus) appliziert. Die resultierende Potenzierung der EPSPs wurde für mindestens 60 Minuten aufgezeichnet.
In den Experimenten zur Prüfung der Wirkung des NHE5-Inhibitors wurde ebenfalls zunächst für 60-120 Minuten die Basislinie aufgenommen. Das Einspülen des NHE5-Inhibitors (10 µM) erfolgte 20 Minuten vor Stimulation. Es wurden wie in den Kontrollexperimenten viermal zwei Doppelpulse mit einem Interpulsabstand der Doppelpulse von 10 ms und einer Breite der Einzelpulse von 0,2 ms im Abstand von 200 ms appliziert. Die Substanz wurde 20 Minuten nach Stimulation ausgewaschen und die Potenzierung des EPSPs für mindestens 60 Minuten aufgezeichnet.

### Ergebnis:

Die Verbindung des Beispiels 1 hatte in der verwendeten Konzentration keine Eigenwirkung auf die synaptische Transmission.
Die Potenzierung nach Gabe des Beispiels 1 betrug 80 min nach Stimulus immer noch 137% der Basislinie, wohingegen die Potenzierung unter Kontrollbedingungen mit 113% der Basislinie schon fast wieder das Niveau der Basislinie erreicht hatte.
Dies zeigt deutlich, dass bereits 10µM der Verbindung des Beispiels 1 die Aufrechterhaltung des schwachen LTP verbessern.

## Patentansprüche

1. Verbindungen der Formel I 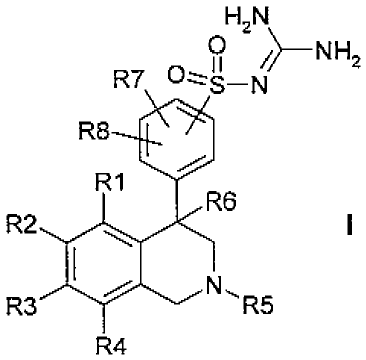 worin bedeuten:
R1 und R3 Wasserstoff;
R2 und R4 unabhängig voneinander Wasserstoff, F, Cl, NH₂, NHCH₃ oder N(CH₃)₂;
R5 Wasserstoff, Methyl, Ethyl oder Cyclopropyl;
R6 Wasserstoff oder Methyl;
R7 und R8 Wasserstoff;
sowie deren pharmazeutisch verträglichen Salze und Trifluoracetate.

2. Verbindungen der Formel I und deren pharmazeutisch verträgliche Salze nach Anspruch 1 zur Verwendung als Medikament.

3. Verwendung einer Verbindung der Formel I und deren pharmazeutisch verträgliche Salze nach Anspruch 1 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls. von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen, zum Einsatz bei Bypassoperationen, zum Einsatz bei Wiederbelebung nach Herzstillstand, oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

4. Verwendung einer Verbindung der Formel I und deren pharmazeutisch verträgliche Salze nach Anspruch 1 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen, zum Einsatz bei Bypassoperationen, zum Einsatz bei Wiederbelebung nach Herzstillstand, oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

5. Verwendung einer Verbindung der Formel I und/oder deren pharmazeutisch verträglicher Salze nach Anspruch 1 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

6. Verwendung einer Verbindung der Formel I und/oder deren pharmazeutisch verträglicher Salze nach Anspruch 1 allein oder in Kombination mit anderen Arzneimitteln oder Wirkstoffen zur Herstellung eines Medikaments zur Behandlung oder Prophylaxe des Schnarchens.

7. Verwendung einer Verbindung der Formel I und/oder deren pharmazeutisch verträglicher Salze nach Anspruch 1 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

8. Verwendung einer Verbindung der Formel I und/oder deren pharmazeutisch verträglicher Salze nach Anspruch 1 allein oder in Kombination mit anderen Arzneimitteln oder Wirkstoffen zur Herstellung eines Medikaments zur Behandlung oder Prophylaxe von Störungen der Darmfunktion.

9. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach Anspruch 1.

10. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach Anspruch 1, in Kombination mit anderen pharmakologischen Wirkstoffen oder Arzneimitteln.

## Claims

1. A compound of the formula I 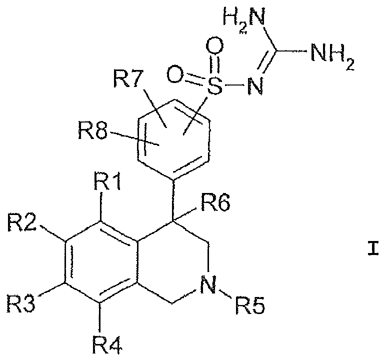 in which:
R1 and R3 are each hydrogen;
R2 and R4 are each independently hydrogen, F, Cl, NH₂, NHCH₃ or N(CH3)2;
R5 is hydrogen, methyl, ethyl or cyclopropyl;
R6 is hydrogen or methyl;
R7 and R8 are each hydrogen;
and also its pharmaceutically acceptable salts and trifluoroacetates.

2. A compound of the formula I and its pharmaceutically acceptable salts as claimed in claim 1 for use as a medicament.

3. The use of a compound of the formula I and its pharmaceutically acceptable salts as claimed in claim 1 for preparing a medicament for the treatment or prophylaxis of disorders of respiratory drive, of respiratory disorders, sleep-related respiratory disorders, sleep apneas, of snoring, of acute and chronic renal disorders, of acute kidney failure and of chronic kidney failure, of disorders of intestinal function, of high blood pressure, of essential hypertension, of disorders of the central nervous system, of disorders resulting from CNS overexcitability, epilepsy and centrally induced convulsions or of states of anxiety, depressions and psychoses, of ischemic states of the peripheral or central nervous system or of stroke, of acute and chronic damage to and disorders of peripheral organs or limbs caused by ischemic events or by reperfusion events, of atherosclerosis, of disorders of lipid metabolism, of thromboses, of disorders of biliary function, of infestation by ectoparasites, of disorders caused by endothelial dysfunction, of protozoal disorders, of malaria, for the preservation and storage of transplants for surgical procedures, for use in surgical operations and organ transplants, for use in bypass operations, in resuscitation after cardiac arrest, or for the treatment of states of shock or of diabetes and late damage from diabetes, or of diseases in which cellular proliferation constitutes a primary or secondary cause, and for maintaining health and prolonging life.

4. The use of a compound of the formula I and its pharmaceutically acceptable salts as claimed in claim 1 in combination with other medicaments or active ingredients for preparing a medicament for the treatment or prophylaxis of disorders of respiratory drive, of respiratory disorders, sleep-related respiratory disorders, sleep apneas, of snoring, of acute and chronic renal disorders, of acute kidney failure and of chronic kidney failure, of disorders of intestinal function, of high blood pressure, of essential hypertension, of disorders of the central nervous system, of disorders resulting from CNS overexcitability, epilepsy and centrally induced convulsions or of states of anxiety, depressions and psychoses, of ischemic states of the peripheral or central nervous system or of stroke, of acute and chronic damage to and disorders of peripheral organs or limbs caused by ischemic events or by reperfusion events, of atherosclerosis, of disorders of lipid metabolism, of thromboses, of disorders of biliary function, of infestation by ectoparasites, of disorders caused by endothelial dysfunction, of protozoal disorders, of malaria, for the preservation and storage of transplants for surgical procedures, for use in surgical operations and organ transplants, for use in bypass operations, in resuscitation after cardiac arrest, or for the treatment of states of shock or of diabetes and late damage from diabetes, or of diseases in which cellular proliferation constitutes a primary or secondary cause, and for maintaining health and prolonging life.

5. The use of a compound of the formula I and/or its pharmaceutically acceptable salts as claimed in claim 1 alone or in combination with other medicaments or active ingredients for preparing a medicament for the treatment or prophylaxis of disorders of respiratory drive and/or of sleep-related respiratory disorders such as sleep apneas.

6. The use of a compound of the formula I and/or its pharmaceutically acceptable salts as claimed in claim 1 alone or in combination with other medicaments or active ingredients for preparing a medicament for the treatment or prophylaxis of snoring.

7. The use of a compound of the formula I and/or its pharmaceutically acceptable salts as claimed in claim 1 alone or in combination with other medicaments or active ingredients for preparing a medicament for the treatment or prophylaxis of acute or chronic renal disorders, of acute kidney failure or of chronic kidney failure.

8. The use of a compound of the formula I and/or its pharmaceutically acceptable salts as claimed in claim 1 alone or in combination with other medicaments or active ingredients for preparing a medicament for the treatment or prophylaxis of disorders of intestinal function.

9. A pharmaceutical preparation for human, veterinary or phytoprotective use, comprising an effective amount of a compound of the formula I and/or of a pharmaceutically acceptable salt thereof as claimed in claim 1.

10. A pharmaceutical preparation for human, veterinary or phytoprotective use, comprising an effective amount of a compound of the formula I and/or of a pharmaceutically acceptable salt thereof as claimed in claim 1, in combination with other pharmacological active ingredients or medicaments.

## Revendications

1. Composés de formule I 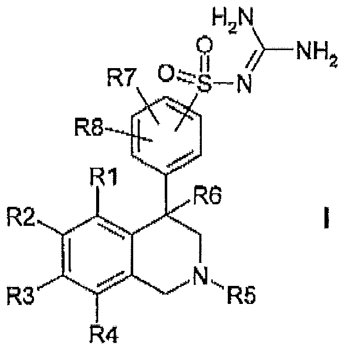 dans laquelle :
R1 et R3 représentent un atome d'hydrogène ;
R2 et R4 représentent indépendamment l'un de l'autre un atome d'hydrogène, F, Cl, NH₂, NHCH₃ ou N(CH₃)₂ ;
R5 représente un atome d'hydrogène, le groupe méthyle, éthyle ou cyclopropyle ;
R6 représente un atome d'hydrogène ou le groupe méthyle ;
R7 et R8 représentent un atome d'hydrogène ;
ainsi que leurs sels pharmaceutiquement acceptables et trifluoroacétates.

2. Composés de formule I et leurs sels pharmaceutiquement acceptables selon la revendication 1, pour utilisation en tant que médicament.

3. Utilisation d'un composé de formule I et de ses sels pharmaceutiquement acceptables selon la revendication 1, pour la fabrication d'un médicament destiné au traitement ou à la prophylaxie de troubles de la commande respiratoire, de troubles respiratoires, de troubles respiratoires dus au sommeil, d'apnées du sommeil, du ronflement, de néphropathies aiguës et chroniques, de l'insuffisance rénale aiguë et de l'insuffisance rénale chronique, de troubles de la fonction intestinale, de l'hypertension artérielle, de l'hypertonie essentielle, de maladies du système nerveux central, de maladies qui résultent d'une hyperexcitabilité du SNC, de l'épilepsie et de convulsions d'origine centrale ou d'états anxieux, de dépressions et de psychoses, d'états ischémiques du système nerveux périphérique ou central ou de l'accident vasculaire cérébral, de lésions et maladies aiguës et chroniques d'organes périphériques ou des membres, qui sont provoquées par des incidents d'ischémie ou par des incidents de reperfusion, de l'athérosclérose, de troubles du métabolisme des lipides, de thromboses, de troubles de la fonction biliaire, de l'attaque par des ectoparasites, de maladies faisant suite à un dysfonctionnement endothélial, de maladies provoquées par des protozoaires, du paludisme, pour la conservation et le stockage de transplants pour des interventions chirurgicales, pour l'utilisation dans des opérations chirurgicales et des greffes d'organes, pour l'utilisation dans des opérations de pontage, pour l'utilisation dans la réanimation après arrêt cardiaque, ou pour le traitement d'états de choc ou du diabète et de complications diabétiques ou de maladies dans lesquelles la prolifération cellulaire représente une cause primaire ou secondaire et pour le maintien de la santé et le prolongement de la vie.

4. Utilisation d'un composé de formule I et de ses sels pharmaceutiquement acceptables selon la revendication 1, en association avec d'autres médicaments ou substances actives pour la fabrication d'un médicament destiné au traitement ou à la prophylaxie de troubles de la commande respiratoire, de troubles respiratoires, de troubles respiratoires dus au sommeil, d'apnées du sommeil, du ronflement, de néphropathies aiguës et chroniques, de l'insuffisance rénale aiguë et de l'insuffisance rénale chronique, de troubles de la fonction intestinale, de l'hypertension artérielle, de l'hypertonie essentielle, de maladies du système nerveux central, de maladies qui résultent d'une hyperexcitabilité du SNC, de l'épilepsie et de convulsions d'origine centrale ou d'états anxieux, de dépressions et de psychoses, d'états ischémiques du système nerveux périphérique ou central ou de l'accident vasculaire cérébral, de lésions et maladies aiguës et chroniques d'organes périphériques ou des membres, qui sont provoquées par des incidents d'ischémie ou par des incidents de reperfusion, de l'athérosclérose, de troubles du métabolisme des lipides, de thromboses, de troubles de la fonction biliaire, de l'attaque par des ectoparasites, de maladies faisant suite à un dysfonctionnement endothélial, de maladies provoquées par des protozoaires, du paludisme, pour la conservation et le stockage de transplants pour des interventions chirurgicales, pour l'utilisation dans des opérations chirurgicales et des greffes d'organes, pour l'utilisation dans des opérations de pontage, pour l'utilisation dans la réanimation après arrêt cardiaque, ou pour le traitement d'états de choc ou du diabète et de complications diabétiques ou de maladies dans lesquelles la prolifération cellulaire représente une cause primaire ou secondaire et pour le maintien de la santé et le prolongement de la vie.

5. Utilisation d'un composé de formule I et/ou de ses sels pharmaceutiquement acceptables selon la revendication 1, seul(s) ou en association avec d'autres médicaments ou substances actives, pour la fabrication d'un médicament destiné au traitement ou à la prophylaxie de troubles de la commande respiratoire et/ou de troubles respiratoires dus au sommeil tels que les d'apnées du sommeil.

6. Utilisation d'un composé de formule I et/ou de ses sels pharmaceutiquement acceptables selon la revendication 1, seul(s) ou en association avec d'autres médicaments ou substances actives, pour la fabrication d'un médicament destiné au traitement ou à la prophylaxie du ronflement.

7. Utilisation d'un composé de formule I et/ou de ses sels pharmaceutiquement acceptables selon la revendication 1, seul(s) ou en association avec d'autres médicaments ou substances actives, pour la fabrication d'un médicament destiné au traitement ou à la prophylaxie de néphropathies aiguës ou chroniques, de l'insuffisance rénale aiguë ou de l'insuffisance rénale chronique.

8. Utilisation d'un composé de formule I et/ou de ses sels pharmaceutiquement acceptables selon la revendication 1, seul(s) ou en association avec d'autres médicaments ou substances actives, pour la fabrication d'un médicament destiné au traitement ou à la prophylaxie de troubles de la fonction intestinale.

9. Préparation pharmaceutique pour l'utilisation humaine, vétérinaire ou phytoprotectrice, contenant une quantité efficace d'un composé de formule I et/ou d'un sel pharmaceutiquement acceptable de celui-ci selon la revendication 1.

10. Préparation pharmaceutique pour l'utilisation humaine, vétérinaire ou phytoprotectrice, contenant une quantité efficace d'un composé de formule I et/ou d'un sel pharmaceutiquement acceptable de celui-ci selon la revendication 1, en association avec d'autres médicaments ou substances actives pharmacologiques.
